# EUROPEAN PATENT SPECIFICATION

(11) **EP 3 908 674 B1**
(45) Date of publication and mention of the grant of the patent: **02.12.2026**
(21) Application number: 19828300.4
(22) Date of filing: 19.12.2019
(51) Int. Cl.: C12Q 1/6834, C12Q 1/6876

(54) **OLIGONUCLEOTIDE DEPOSITION ONTO POLYPROPYLENE SUBSTRATES**
ABSCHEIDUNG VON OLIGONUCLEOTIDEN AUF POLYPROPYLENSUBSTRATEN
DÉPÔT DES OLIGONUCLÉOTIDES SUR DES SUBSTRATES POLYPROPYLENE

(30) Priority: 08.01.2019 GB 201900262
(43) Date of publication of application: 17.11.2021
(73) Proprietor: Hunan Sanway Quinten Biotech Co., Ltd., Changsha, Hunan 410205 (CN)
(72) Inventor: HANNANT, Jennifer, Newcastle upon Tyne Tyne and Wear NE1 2JQ (GB); FLEMING, Jodie Leanne, Newcastle upon Tyne Tyne and Wear NE1 2JQ (GB)
(74) Representative: Murgitroyd & Company
(86) International application number: PCT/GB2019/053633
(87) International publication number: WO 2020/144454

(56) References cited:
- WO-A2-00/70088
- US-A1- 2001 018 513
- P. KUMAR ET AL: "A Rapid Method for the Construction of Oligonucleotide Arrays", BIOCONJUGATE CHEMISTRY, vol. 14, no. 3, 1 May 2003 (2003-05-01), pages 507 - 512, XP055084967, ISSN: 1043-1802, DOI: 10.1021/bc025646q
- GUBALA V ET AL: "Functionalization of cyclo-olefin polymer substrates by plasma oxidation: Stable film containing carboxylic acid groups for capturing biorecognition elements", COLLOIDS AND SURFACES. B, BIOINTERFACES, ELSEVIER, AMSTERDAM, NL, vol. 81, no. 2, 1 December 2010 (2010-12-01), pages 544 - 548, XP027288718, ISSN: 0927-7765, [retrieved on 20100803]
- SITUMA C ET AL: "Merging microfluidics with microarray-based bioassays", BIOMOLECULAR ENGINEERING, ELSEVIER, NEW YORK, NY, US, vol. 23, no. 5, 1 October 2006 (2006-10-01), pages 213 - 231, XP028041926, ISSN: 1389-0344, [retrieved on 20061001], DOI: 10.1016/J.BIOENG.2006.03.002
- SITUMA C ET AL: "Fabrication of DNA microarrays onto poly(methyl methacrylate) with ultraviolet patterning and microfluidics for the detection of low-abundant point mutations", ANALYTICAL BIOCHEMISTRY, ELSEVIER, AMSTERDAM, NL, vol. 340, no. 1, 1 May 2005 (2005-05-01), pages 123 - 135, XP022593929, ISSN: 0003-2697, [retrieved on 20050328], DOI: 10.1016/J.AB.2005.01.044

## Description

### Technical Field

The present invention relates to a method of immobilising an oligonucleotide on a polypropylene surface. The invention has utility in biomolecular assays, and in particular in hybridisation techniques where it can be used to attach single-stranded deoxyribonucleic acid (ssDNA) probes to the surface of polypropylene components such as microfluidic cassettes and plugs.

### Background

Microfluidic cassettes, or microfluidic flow cells, have a microfluidic structure in which one or more fluid channels are formed. The fluid channel typically comprises a flowable carrier medium, with which reagents, housed in microchannels or chambers, are brought into contact for the purposes of analysis or testing. In most microfluidic systems, it is necessary to optimise flow of the carrier through the channels to ensure efficient throughput. This can be manipulated by careful selection of the material making up the microfluidic flow cell or component thereof.

While traditionally composed of silicon, and then glass, the use of polymeric materials in microfluidic applications has increased in recent years.

Kumar et al., Bioconjugate Chemistry, Vol. 14(3); 2003, pp. 507-512, discloses a method for the construction of oligonucleotide arrays on surfaces. The method involves the generation of hydroxyl functionalities on commonly used surfaces for construction of oligonucleotide arrays followed by their activation with trifluoroethanesulfonyl chloride (tresyl chloride). The activated surface in the subsequent reaction is used to covalently immobilise oligonucleotides in regioselective fashion to create an oligonucleotide array.

Gubala et al., Colloids and Surfaces B, Biointerfaces, Vol. 81(2); 2010; pp. 544-548, discloses direct immobilisation of biomolecules on plasma oxidized cyclo-olefin polymers (COP).

Situma et al., Biomolecular Engineering, Vol. 23 (5); 2006; pp. 213-231 provides an overview of the coupling of DNA, protein and cell microarrays to microfluidics and discusses potential improvements required for the implementation of these technologies into biomedical and clinical applications.

WO00/70088 A2 discloses a method of attaching unmodified biopolymers, particularly unmodified polynucleotides, directly to a solid support.

US2001/018513 A1 discloses a method for extracting nucleic acids from a biological material such as blood, comprising contacting the mixture with a material at a pH such that the material is positively charged and will bind negatively charged nucleic acids, and then eluting the nucleic acids at a pH when the said materials possess a neutral or negative charge to release the nucleic acids.

Situma et al., Analytical Biochemistry, Vol. 340; 2005; pp. 123-135 discloses the fabrication of DNA microarrays onto poly(methyl methacrylate) with ultraviolet patterning and microfluidics for the detection of low-abundant point mutations.

For many applications, polycarbonate is now the preferred material for microfluidic applications involving biomaterials. The use of polycarbonate in microfluidic applications strikes a balance between flowability, and the ability to immobilise reagents on its surface.

Polypropylene is an inert, non-polar material which exhibits excellent flow characteristics. In addition, the low background fluorescence of polypropylene makes it well suited for techniques involving fluorescence detection. However, the inherent inert nature of polypropylene makes immobilisation on its surface difficult, thereby limiting its use in microfluidic assays where attachment of reagents such as biomolecules, is often required. Hybridisation assays, for instance, typically require the flow of a medium over an immobilised single-stranded oligonucleotide, such as single-stranded DNA (ssDNA). Immobilisation of the ssDNA must be sufficiently stable to ensure that the strands of DNA remain attached to the surface during the performance of the assay, and must allow selective and sensitive detection in a reproducible manner. In addition, the immobilisation technique itself should be efficient and not require vast quantities or excesses of expensive materials, such as labelled biomolecules. To date, such immobilisation has proven challenging.

"Oligonucleotide" means a molecule which comprises a chain of nucleotides. Oligonucleotides are commonly in the form of DNA or ribonucleic acid (RNA). The term "oligonucleotide" includes polynucleotides of genomic DNA or RNA, complementary DNA (cDNA), and polynucleotides of semisynthetic or synthetic origin. Standard nucleotide bases may also be substituted with nucleotide isoform analogs. In this context the term "oligonucleotide" is somewhat interchangeable with "polynucleotide", although it generally refers to nucleotide chains of shorter length.

It is an object of the present invention to obviate or mitigate one or more of these problems, and in aspects of the invention, to provide an improved method of immobilising an oligonucleotide onto a polypropylene surface.

### Summary of the Invention

The present invention relates to a method of immobilizing an oligonucleotide on a polypropylene surface, the method comprising oxidising at least a portion of the polypropylene surface; and bringing an amine-terminated oligonucleotide into contact with the oxidised polypropylene to immobilise the oligonucleotide to the oxidised polypropylene surface via the formation of imine bonds.

The invention also relates to a microfluidic cassette or portion thereof, e.g. a plug insertable to form part of a microfluidic channel, having an oligonucleotide immobilised thereon by the method of the invention.

Various further features and aspects of the invention are defined in the claims.

### Brief Description of the Drawings

Embodiments of the present invention will now be described by way of example only with reference to the accompanying drawings where like parts are provided with corresponding reference numerals and in which:

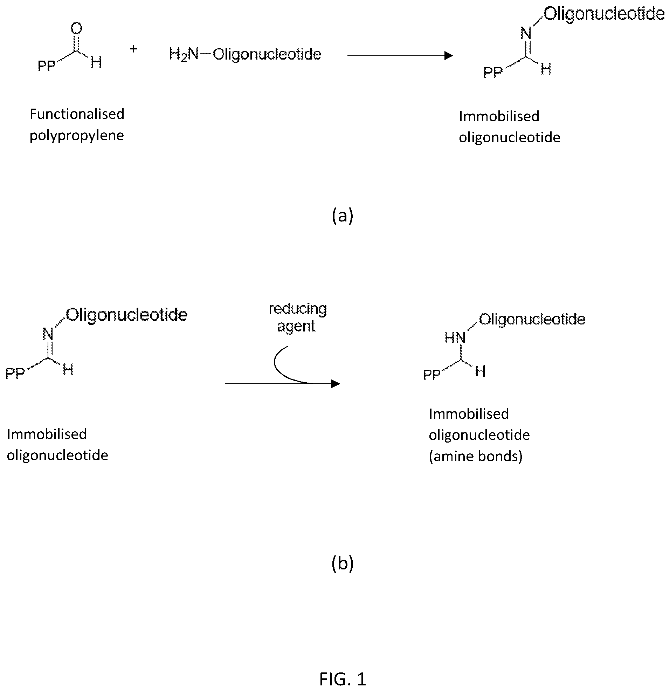

Figure 1(a) illustrates the reaction of an amine-terminated oligonucleotide with an activated polypropylene surface: the amine-terminated oligonucleotide reacts with aldehyde groups on the activated polypropylene surface to form imine bonds; which, in an optional step shown in Figure 1(b) can subsequently be converted to amine bonds via the use of a reducing agent.

### Detailed Description

The present invention relates to a method of immobilizing an oligonucleotide on a polypropylene surface, the method comprising oxidising at least a portion of the polypropylene surface; and bringing an amine-terminated oligonucleotide into contact with the oxidised polypropylene surface to immobilise the oligonucleotide to the oxidised polypropylene surface via the formation of imine bonds.

According to the present invention, at least a portion of the polypropylene surface is oxidised. The polypropylene surface may be, for example, an internal or external surface of a cassette, slide, plate or any component of a microfluidic assay. As would be understood by one skilled in the art, in this embodiment, immobilisation of the oligonucleotide onto a portion of the surface only would be required, in order to allow the reagent to flow over the polypropylene surface and bind or react selectively with the immobilised oligonucleotide, such as in a hybridisation assay.

In an embodiment, the oxidising step is carried out using plasma activation, flame treatment, chemical oxidation or UV-ozone.

The plasma activation may be arc discharge, corona discharge, dielectric barrier discharge or piezoelectric direct discharge.

In an embodiment, the oxidising step is carried out using a low-pressure oxygen plasma system.

The parameters for performing the low-pressure oxygen plasma activation would be readily ascertained by one skilled in the art; however, typically the process is carried out for from 1 minute to 15 minutes, with 70 to 100% generator power; with a flow rate of from 2 sccm to 20 sccm, or from 2 sccm to 10 sccm oxygen, or typically 5 sccm.

Typically, the oxygen plasma activation generates multiple oxygen-containing groups such as aldehyde groups, on the polypropylene surface, which can react with the amine-terminated oligonucleotide to form imine bonds. Advantageously, when oxygen plasma is used for functionalisation of the polypropylene surface, typically only oxygen-containing groups are generated, thereby minimising undesirable side-reactions which can result from the generation of other functional groups, such as nitrogen-containing groups which can result from the use of ammonia plasma. Oxygen plasma is also readily available and inexpensive, making it particular suitable for use in the method of the present invention. Oxygen plasma generates a relatively high concentration of aldehyde groups on the polypropylene surface, making it particularly suitable for use in the present invention.

Alternatively, the oxidising step can be carried out using chemical oxidation. Suitable chemical oxidation systems include chromium oxide, sodium dichromate, sulphuric acid, fuming nitric acid, potassium permanganate solution, ammonium peroxydisulphate solution, acid piranha solution, base piranha solution, methanol/hydrochloric acid; and combinations thereof.

In an embodiment, chemical oxidation can be performed using sodium dichromate and sulphuric acid.

According to the invention, the amine-terminated oligonucleotide is brought into contact with the oxidised propylene surface. The amine-terminated oligonucleotide is brought into contact with the oxidised propylene surface under conditions sufficient to immobilise the oligonucleotide to the oxidised polypropylene surface via the formation of imine bonds.

The amine-terminated oligonucleotide may be a labelled or unlabelled oligonucleotide.

In an embodiment, the oligonucleotide is single-stranded DNA (ssDNA).

In an embodiment, the ssDNA is a labelled DNA probe. Optionally, the labelled DNA probe is a fluorescent-labelled DNA probe.

For instance, the immobilised oligonucleotide can be amine-terminated ssDNA which can be incorporated in a hybridisation assay as a probe for target DNA. The ssDNA can be labelled, such as, for example, with a fluorescent cyanine dye, or a ruthenium dye. Polypropylene has low background fluorescence, and therefore the immobilisation of fluorescent labelled biomolecules to a polypropylene surface is particularly advantageous.

The terminal amine group may be a 5' amine group or a 3' amine group. In an embodiment, the terminal amine group is a 5' amine group. Amine-terminated oligonucleotide can be purchased commercially, or can be prepared using known methods.

The step of bringing the amine-terminated oligonucleotide into contact with the oxidised polypropylene surface is carried out under conditions sufficient to immobilise the amine-terminated oligonucleotide via the formation of imine bonds with oxygen-containing functional groups on the activated polypropylene surface. Such conditions would be apparent to one skilled in the art, or could be optimised using known techniques.

A method of immobilising an oligonucleotide on a polypropylene surface according to the method of the present invention is illustrated schematically in Figure 1(a), and shows reaction of aldehyde groups on the activated polypropylene surface with amine-terminated ssDNA to give immobilised ssDNA on the polypropylene surface via the formation of imine bonds.

For example, the amine-terminated nucleotide may be brought into contact with the oxidised polypropylene surface in the presence of a buffer.

Suitable buffers would be known to one skilled in the art and include, for example, 2-(N-morpholino)ethanesulfonic acid (MES), 3-(N-morpholino)propanesulfonic acid (MOPS),saline-sodium citrate (SSC), 2,2-Bis(hydroxymethyl)-2,2',2"-nitrilotriethanol (BIS-TRIS), Piperazine-N,N'-bis(2-ethanesulfonic acid) (PIPES), 3-morpholinopropanesulfonic acid (MOPSO), 1,3-Bis[tris(hydroxymethyl)methylamino]propane (BIS-TRIS propane), N,N-Bis(2-hydroxyethyl)-2-aminoethanesulfonic acid, N,N-Bis(2-hydroxyethyl)taurine (BES), 3-[(3-Cholamidopropyl)dimethylammonio]-1-propanesulfonate hydrate (CHAPS), and phosphate buffers.

In an embodiment, the buffer is 2-(N-morpholino)ethanesulfonic acid (MES).

In an embodiment, for instance, the amine-terminated oligonucleotide may be printed onto the oxidised polypropylene surface. The printed surface may be allowed to incubate for a period of from less than 1 hour to 14 days, e.g. from 5 minutes to 14 days, from 5 minutes to 10 days. Preferably the incubation is allowed to take place for from 5 minutes to 24 hours. Incubation may take place under ambient temperature and humidity conditions. If a fluorescent probe is being used, the printed surfaces may be kept in darkness during the incubation period.

In an embodiment of the invention, the polypropylene surface is an internal surface of a microfluidic channel in a microfluidic cassette for use in a hybridisation assay or a surface of a plug/insert that forms part of a microfluidic channel. The ssDNA-immobilised polypropylene surface may be used in a hybridisation assay.

It will be understood that the immobilisation of an oligonucleotide onto the internal surface can occur during the fabrication of a microfluidic cassette.

In an embodiment, the method further comprises the step of blocking the oligonucleotide-immobilised surface with a blocking agent.

According to this embodiment of the invention, non-specific binding of other biomolecules or other components of the assay to the activate polypropylene surface during performance of the assay can be minimised by blocking unoccupied binding sites with a blocking reagent. Any suitable blocking reagent, i.e. one which can reduce or eliminate non-specific binding, which does not participate in the reactions that form part of the assay; and which does not otherwise damage components of the assay, can be used.

In addition, as shown schematically in Figure 1(b), the ssDNA immobilisation (via imine bonds) is unstable. When a suitable reducing agent is used, the imine bonds of the immobilised ssDNA are converted to amine bonds, leading to a more stable immobilisation.

Blocking/reducing agents which could be used include alkali and alkaline earth metal borohydrides such as sodium borohydride and lithium borohydride; aluminium borohydride, zinc borohydride, sodium cyanoborohydride, sodium triacetoxyborohydride, and acidic solutions of Zn²⁺ or Fe²⁺.

In an embodiment, the blocking/reducing agent is sodium borohydride.

In an embodiment, the method further comprises one or more washing steps. The washing steps may be performed prior to the blocking step. A detergent, such as sodium dodecyl sulfate (SDS) may be used in the washing step.

### Examples:

The following examples are intended to be illustrative only and are not intended to limit the scope of the invention.

### Example 1.1: Amine-terminated ssDNA

Unlabelled DNA probes modified on the 5' end with an amine termination, a C₁₂ linker and 2 hexaethylene glycol spacer modifications were purchased from Integrated DNA Technologies (Illinois, USA).

Cy5 and Cy3 labelled DNA probes, modified on the 5' end with an amine termination and a C₆ linker were purchased from Integrated DNA Technologies (Illinois, USA).

### Example 1.2 Preparation of DNA ink

A DNA ink for deposition was prepared as follows:

### 1.2.1 Preparation of MES buffer

A 0.1 M MES (w/0.9% NaCl, pH 4.6) buffer solution was prepared as follows: 1.066 g of MES monohydrate was weighed into a 50 ml falcon tube. 45 ml of ultrapure water was added from a measuring cylinder. 0.450 g of NaCl was weighed in a weighing boat and added to the MES solution, which was shaken until complete dissolution occurred. The pH of the solution was measured, and adjusted to pH 4.6 using a 0.1 M or 1 M NaOH solution prepared from NaOH pellets (Sigma-Aldrich, product # 221465). The remaining volume of ultrapure water needed to make up to 50 ml was added via pipette. The final pH was measured to confirm it fell within the range of pH 4.55 and pH 4.65. The solution was filtered through a 0.22 µm filter into a clean falcon tube. The buffer was stored in a fridge before use (up to 1 week).

### 1.2.2 MES buffer containing 1% glycerol

When the solution was required, 160 µl of 0.1 M MES buffer (section 1.2.1) was pipetted into an Eppendorf tube. 2 µl of glycerol was pipetted into the solution, followed by 18 µl of ultrapure water which was pipetted into the solution.

### 1.2.3 Preparation of DNA Ink

### 1.2.3.1 20 µl 100 µM (unlabelled) DNA probe solution

A 20 µl 100 µM (unlabelled) DNA probe solution was prepared as follows:
18 µl of the MES buffer with 1% glycerol solution prepared in section 1.2.2 was pipetted into an Eppendorf tube. 2 µl of 1 mM unlabelled DNA probe was pipetted into the solution.

### 1.2.3.2 20 µl 50 µM (labelled) DNA probe solution

A 20 µl 50 µM (labelled) DNA probe solution was prepared as follows:
18 µl of the MES buffer with 1% glycerol solution prepared in section 1.2.2 was pipetted into an Eppendorf tube. 1 µl of ultrapure water was pipetted into the solution and then 1 µl of 1 mM Cy5 or Cy3-labelled DNA probe was pipetted into the solution.

### Example 1.3: Activation of polypropylene surface

### 1.3.1 Pre-cleaning of PP surface

Polypropylene (PP) slides and polypropylene array plugs were rinsed with isopropyl alcohol (IPA) from a squeezy bottle at a minimum of 1 ml IPA per cm² of PP surface, and then dried using a stream of nitrogen until all visible traces of IPA had disappeared. The slides were then inserted into a glass holder and baked in the oven at 70 °C, atmospheric pressure, for 15 minutes.

Although the PP surface was pre-cleaned in this specific example, the cleaning step is optional, and subsequent testing has confirmed that it is not an essential step.

### 1.3.2 Activation of polypropylene surface

The clean PP samples (slides and array plugs) were placed into a plasma asher (Diener^{™} Pico, Serial number 116299), with the PP surface to which immobilisation is required facing upwards. The plasma asher generator power was set to 90%, the O₂ flow rate to 5 sccm, and the PP surface was activated for 15 minutes. Samples were then removed from the asher and placed in the printer chamber (Scienion SX, Berlin, Germany) until needed. All activated samples were used within 24 hours of activation.

### Example 1.4 DNA Deposition

10 minutes after plasma activation, the DNA ink solutions prepared in example 1.2.3 above were deposited onto the activated PP surfaces using a Scienion sciFLEXARRAYER SX printer (Scienion SX, Berlin, Germany). The humidity was set to 65% (cold mist), and the inks were printed with 2 drops per spot with a drop volume of between 200 pl and 220 pl. Immediately after deposition, the PP slides and plugs were placed into a clean dish or tray, wrapped in foil, and left to incubate at ambient temperature and humidity for between 6 and 8 days.

### Example 1.5 Blocking

A blocking solution was prepared by adding 15 ml 10x PBS, 135 ml ultrapure water and 50 ml ethanol to a 500 ml Duran bottle. 0.5 g of sodium borohydride (Sigma, product code 213462) was added to the solution. The solution was stirred for 15 minutes.

Five reservoirs were prepared with 1). 0.2% SDS, 2) ultrapure water; 3) the blocking solution prepared above; 4) 0.2% SDS and 5) ultrapure water. Immobilised slides and plugs prepared above were placed into the first reservoir, left for 2 minutes, then agitated by lifting up and down before being left in the reservoir for a further two minutes. The slides and plugs were then placed into the second reservoir containing clean ultrapure water, in a similar fashion (i.e. being left for 2 minutes, agitated, then left for 2 minutes). The slides and plugs were then placed into the third reservoir containing the blocking solution where they were left for 15 minutes. The slides and plugs were then placed into the fourth solution, i.e. a clean 0.2% SDS solution where they were left for 2 minutes, then agitated by lifting up and down, before being left in the reservoir for two minutes, and then they were placed into the fifth reservoir, which contained fresh ultrapure water, where they were again, left for 2 minutes, then agitated by lifting up and down, before being left in the reservoir for two minutes. The slides and plugs were then dried using nitrogen. The plugs were not allowed to dry between each step, and clean solutions were used for each slide/plug.

### Example 1.6 Warfarin assay

A warfarin assay was printed and blocked using the procedure outlined above. Three array plugs were hybridised with a PCR amplified swab sample. This swab sample is wild type homozygous in the *2 SNP. Fluorescence values of the hybridised array plugs imaged at 100 ms exposure are shown in Table 1. Discrimination factors were calculated and were in the ranges expected for a *2 wild type sample.

**Table 1: median fluorescence intensity of each probe type on each of the three arrays**

| | Array plug 1 | Array plug 2 | Array plug 3 |
|---|---|---|---|
| Alignment control | 13,422 | 13,087 | 12,075 |
| CYP2C9*2 wildtype | 28,022 | 28,401 | 25,919 |
| CYP2C9*2 mutant | 1,647 | 1,523 | 1,399 |
| Non-specific control | -126 | -33 | -216 |

Results demonstrate that the immobilisation technique according to the invention allows for selective and sensitive detection to take place, with minimal background signal for fluorescence measurements. Surprisingly, no coupling agent was required to instigate binding between the oxygen-containing functional groups on the activated polypropylene surface and the amine-terminated ssDNA, and sufficient binding was achieved to allow for reproducible measurements to take place.

The method of the invention therefore represents a convenient technique for the immobilisation of amine-terminated oligonucleotides onto polypropylene, using readily available instrumentation and reagents, and which results in sufficient reproducible immobilisation for use in hybridisation techniques.

All of the features disclosed in this specification (including any accompanying claims, abstract and drawings), and/or all of the steps of any method or process so disclosed, may be combined in any combination, except combinations where at least some of such features and/or steps are mutually exclusive. Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features. The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations).

## Claims

1. A method of immobilizing an oligonucleotide on a polypropylene surface, the method comprising:
oxidising at least a portion of the polypropylene surface;
and
bringing an amine-terminated oligonucleotide into contact with the oxidised polypropylene surface to immobilise the oligonucleotide to the oxidised polypropylene surface, wherein the amine-terminated oligonucleotide is immobilised to the oxidised polypropylene surface via the formation of imine bonds.

2. A method as claimed in claim 1, wherein the oxidising step is carried out using plasma activation, flame treatment, chemical oxidation or UV-ozone.

3. A method as claimed in claim 2, wherein the oxidising step is carried out using oxygen plasma.

4. A method as claimed in any preceding claim, wherein the oligonucleotide is single-stranded DNA (ssDNA).

5. A method as claimed in claim 4, where the ssDNA is a labelled DNA probe, optionally a fluorescent-labelled DNA probe.

6. A method as claimed in any preceding claim, wherein the step of bring the amine-terminated oligonucleotide into contact with the oxidised polypropylene surface is performed in the presence of a buffer.

7. A method as claimed in claim 6, wherein the buffer is selected from 2-(N-morpholino)ethanesulfonic acid (MES), 3-(N-morpholino)propanesulfonic acid (MOPS) and saline-sodium citrate (SSC), 2,2-Bis(hydroxymethyl)-2,2',2"-nitrilotriethanol (BIS-TRIS), Piperazine-N,N'-bis(2-ethanesulfonic acid) (PIPES), 3-morpholinopropanesulfonic acid (MOPSO), 1,3-
Bis[tris(hydroxymethyl)methylamino]propane (BIS-TRIS propane), N,N-Bis(2-hydroxyethyl)-2-aminoethanesulfonic acid, N,N-Bis(2-hydroxyethyl)taurine (BES), 3-[(3-Cholamidopropyl)dimethylammonio]-1-propanesulfonate hydrate (CHAPS), and phosphate buffers.

8. A method as claimed in any preceding claim, wherein the polypropylene surface is a surface of a plug or cassette for a hybridisation array, or a surface of a slide.

9. A method as claimed in any preceding claim, wherein the method further comprises the step of reducing the imine bonds to amine bonds with a reducing agent.

10. A method as claimed in claim 9, wherein the reducing agent is sodium borohydride.

## Patentansprüche

1. Ein Verfahren zum Immobilisieren eines Oligonukleotids auf einer Polypropylenoberfläche, wobei das Verfahren Folgendes beinhaltet:
Oxidieren mindestens eines Teils der Polypropylenoberfläche;
und
In-Kontakt-Bringen eines aminoterminierten Oligonukleotids mit der oxidierten Polypropylenoberfläche, um das Oligonukleotid an der oxidierten Polypropylenoberfläche zu immobilisieren, wobei das aminoterminierte Oligonukleotid über die Bildung von Iminbindungen an der oxidierten Polypropylenoberfläche immobilisiert wird.

2. Verfahren gemäß Anspruch 1, wobei der Oxidationsschritt unter Verwendung von Plasmaaktivierung, Flammenbehandlung, chemischer Oxidation oder UV-Ozon durchgeführt wird.

3. Verfahren gemäß Anspruch 2, wobei der Oxidationsschritt unter Verwendung von Sauerstoffplasma durchgeführt wird.

4. Verfahren gemäß einem der vorhergehenden Ansprüche, wobei das Oligonukleotid einzelsträngige DNA (ssDNA) ist.

5. Verfahren gemäß Anspruch 4, wobei die ssDNA eine markierte DNA-Sonde, gegebenenfalls eine fluoreszenzmarkierte DNA-Sonde, ist.

6. Verfahren gemäß einem der vorhergehenden Ansprüche, wobei der Schritt des In-Kontakt-Bringens des aminoterminierten Oligonukleotids mit der oxidierten Polypropylenoberfläche in Gegenwart eines Puffers durchgeführt wird.

7. Verfahren gemäß Anspruch 6, wobei der Puffer ausgewählt ist aus 2-(N-Morpholino)ethansulfonsäure (MES), 3-(N-Morpholino)propansulfonsäure (MOPS) und Saline-Natriumcitrat (SSC), 2,2-Bis(hydroxymethyl)-2,2',2"-nitrilotriethanol (BIS-TRIS), Piperazin-N,N'-bis(2-ethansulfonsäure) (PIPES), 3-Morpholinopropansulfonsäure (MOPSO), 1,3-Bis[tris(hydroxymethyl)methylamino]propan (BIS-TRIS-Propan), N,N-Bis(2-hydroxyethyl)-2-aminoethansulfonsäure, N,N-Bis(2-hydroxyethyl)taurin (BES), 3-[(3-Cholamidopropyl)dimethylammonio]-1-propansulfonathydrat (CHAPS) und Phosphatpuffern.

8. Verfahren gemäß einem der vorhergehenden Ansprüche, wobei die Polypropylenoberfläche eine Oberfläche eines Stopfens oder einer Kassette für ein Hybridisierungsarray oder eine Oberfläche eines Objektträgers ist.

9. Verfahren gemäß einem der vorhergehenden Ansprüche, wobei das Verfahren ferner den Schritt des Reduzierens der Iminbindungen zu Aminbindungen mit einem Reduktionsmittel beinhaltet.

10. Verfahren gemäß Anspruch 9, wobei das Reduktionsmittel Natriumborhydrid ist.

## Revendications

1. Un procédé d'immobilisation d'un oligonucléotide sur une surface en polypropylène, le procédé comprenant :
l'oxydation d'au moins une portion de la surface en polypropylène ;
et
la mise en contact d'un oligonucléotide à terminaison amine avec la surface en polypropylène oxydée afin d'immobiliser l'oligonucléotide sur la surface en polypropylène oxydée, dans lequel l'oligonucléotide à terminaison amine est immobilisé sur la surface en polypropylène oxydée au moyen de la formation de liaisons imine.

2. Un procédé tel que revendiqué dans la revendication 1, dans lequel l'étape d'oxydation est effectuée en utilisant une activation par plasma, un traitement à la flamme, une oxydation chimique ou un traitement UV-ozone.

3. Un procédé tel que revendiqué dans la revendication 2, dans lequel l'étape d'oxydation est effectuée en utilisant un plasma d'oxygène.

4. Un procédé tel que revendiqué dans n'importe quelle revendication précédente, dans lequel l'oligonucléotide est de l'ADN simple brin (ADNsb).

5. Un procédé tel que revendiqué dans la revendication 4, où l'ADNsb est une sonde d'ADN marquée, facultativement une sonde d'ADN marquée par fluorescence.

6. Un procédé tel que revendiqué dans n'importe quelle revendication précédente, dans lequel l'étape de mise en contact de l'oligonucléotide à terminaison amine avec la surface en polypropylène oxydée est réalisée en présence d'un tampon.

7. Un procédé tel que revendiqué dans la revendication 6, dans lequel le tampon est sélectionné parmi l'acide 2-(N-morpholino)éthanesulfonique (MES), l'acide 3-(N-morpholino)propanesulfonique (MOPS) et le citrate de sodium salin (SSC), le 2,2-bis(hydroxyméthyl)-2,2',2"-nitrilotriéthanol (BIS-TRIS), l'acide pipérazine-N,N'-bis(2-éthanesulfonique) (PIPES), l'acide 3-morpholinopropanesulfonique (MOPSO), le 1,3-bis[tris(hydroxyméthyl)méthylamino]propane (BIS-TRIS propane), l'acide N,N-bis(2-hydroxyéthyl)-2-aminoéthanesulfonique, la N,N-bis(2-hydroxyéthyl)taurine (BES), l'hydrate de 3-[(3-cholamidopropyl)diméthylammonio]-1-propanesulfonate (CHAPS), et des tampons phosphate.

8. Un procédé tel que revendiqué dans n'importe quelle revendication précédente, dans lequel la surface en polypropylène est une surface d'un bouchon ou d'une cassette pour un réseau d'hybridation, ou une surface d'une lame.

9. Un procédé tel que revendiqué dans n'importe quelle revendication précédente, le procédé comprenant en outre l'étape de réduction des liaisons imine en liaisons amine à l'aide d'un agent réducteur.

10. Un procédé tel que revendiqué dans la revendication 9, dans lequel l'agent réducteur est le borohydrure de sodium.
